# EUROPÄISCHE PATENTSCHRIFT

(11) **EP 2 080 918 B1**
(45) Veröffentlichungstag und Bekanntmachung des Hinweises auf die Patenterteilung: **03.10.2012**
(21) Anmeldenummer: 09450008.9
(22) Anmeldetag: 13.01.2009
(51) Int. Cl.: F16C 1/04, F16C 1/06, A61B 17/16

(54) **Flexible Antriebswelle**
Flexible drive shaft
Arbre d'entraînement flexible

(30) Priorität: 15.01.2008 AT 262008 U
(43) Veröffentlichungstag der Anmeldung: 22.07.2009
(73) Patentinhaber: IMA Integrated Microsystems Austria GmbH, 2700 Wr. Neustadt (AT)
(72) Erfinder: Weissenböck, Herbert, 2873 Feistritz am Wechsel (AT); Kment, Christoph, 1130 Wien (AT); Kölndorfer, Jürgen, 1100 Wien (AT)
(74) Vertreter: Haffner und Keschmann Patentanwälte KG

(56) Entgegenhaltungen:
- EP-A- 0 986 989
- WO-A-01/22889
- WO-A2-2007/039875
- BE-A- 517 887
- DE-A1- 1 775 442
- US-A- 5 085 283
- US-A1- 2007 093 840

## Beschreibung

Die Erfindung betrifft ein handgeführtes chirurgisches Arbeitsgerät mit einem Rotationsantrieb, einer mit dem Rotationsantrieb verbundenen, in einem gekrümmten, vorzugsweise biegsamen Rohr rotierbar geführten flexiblen Antriebswelle und einem mit dem abtriebsseitigen Ende der Antriebswelle verbundenen Rotationskopf, wobei die flexible Antriebswelle von einem antriebsseitigen und einem abtriebsseitigen Endstück und einer Mehrzahl von zwischen den beiden Endstücken (2,3) in Abstand voneinander angeordneten Segmenten (4) gebildet ist, die mit Hilfe von flexiblen Verbindungselementen (5,6,7,8) untereinander und mit den Endstücken (2,3) zur Übertragung der Rotationsbewegung verbunden sind. Ein derartiges Handgerät ist der US 2007/0093840 Al zu entnehmen.

Flexible bzw. biegsame Antriebswellen werden verwendet, wenn das anzutreibende Element beweglich ist und die Antriebsquelle nicht mitbewegt werden kann oder soll, oder wenn das zu bewegende Objekt nicht auf direktem Weg erreicht werden kann. Flexible Antriebswellen kommen beispielsweise bei handgeführten Arbeitsgeräten, wie z.B. bei chirurgischen Instrumenten zum Einsatz, wo ein Werkzeugkopf zu entsprechender Rotation angetrieben werden soll. Es kann sich hierbei um Bohr- oder Schleifköpfe oder dergleichen handeln.

Es existiert eine Reihe von Vorschlägen zur Kraftübertragung in gekrümmten Hohlwellen, wobei diese Systeme üblicherweise auf einer Spiralfeder basieren, die in einem gleichfalls aus einer Federstruktur ausgebildeten Außenmantel rotieren kann. Durch die Federstruktur ergibt sich bei Rotationskräften stets eine meist unerwünschte Federwirkung, wodurch sich die Antriebswelle bzw. die Antriebsfeder in ihrem Durchmesser verändern kann, was zu erhöhter Reibung an der Außenmantelwand führen kann. Eine verbesserte Ausführung einer flexiblen Welle zur Kraftübertragung ist aus der US 6,533,749 bekannt, wo durch zwei ineinander gefügte Flachfederstrukturen der erwähnte Nachteil der Federwirkung bzw. Verformung bei Belastung verhindert werden soll. Die dort beschriebene Konstruktion führt durch die großen Gleitflächen der Flachfederstruktur aber zu verstärkten Problemen durch Reibungswärme.

In der EP 986 989 Al wird eine weitere Ausführung einer flexiblen Antriebswelle beschrieben, bei der mäanderförmige Einschnitte abwechselnd Zähne und Einbuchtungen von Wellensegmenten definieren, wobei in jeder Einbuchtung wiederum ein Zahn angeordnet ist und jeder Zahn in einer Einbuchtung angeordnet ist. Dieses System ist jedoch außerordentlich reibungsbehaftet und führt daher zu einer großen Wärmeentwicklung.

Die vorliegende Erfindung zielt darauf ab, ein Handgerät mit einer flexiblen Antriebswelle zu schaffen, mit welcher große Drehmomente übertragen werden können und welche auch für hohe Umdrehungszahlen im Bereich von mehreren 1000 Umdrehungen pro Minute geeignet ist. Zudem sollen die mit den herkömmlichen Systemen verbundenen negativen Auswirkungen von Federstrukturen und dergleichen vermieden und Reibungsverluste möglichst herabgesetzt werden. Weiters soll die Möglichkeit geschaffen werden, Medien wie etwa Kühlgas, Spülmittel und dergleichen durch die flexible Welle zu transportieren.

Zur Lösung dieser Aufgabe zeichnet sich das erfindungsgemäße Handgerät im Wesentlichen dadurch aus, dass die Segmente jeweils eine in der Wellenachse liegende Durchführung aufweisen, in welcher ein flexibler Schlauch für den Transport von Medien, wie z.B. Kühlgas, Spülmedium, Schmiermedium oder Pharmazeutika angeordnet ist, wobei die Medien dem Rotationskopf zugeführt sind. Dadurch, dass die flexible Welle von mehreren in Abstand voneinander angeordneten Segmenten gebildet ist, die vorzugsweise jeweils aus einem im Wesentlichen unnachgiebigen Material bestehen, die mit Hilfe von flexiblen Verbindungselementen untereinander verbunden sind, entsteht insgesamt ein biegsames System, das hohe Drehmomente übertragen kann. Die Biegsamkeit der Welle wird hierbei auf Grund der flexiblen Verbindungselemente ermöglicht, wobei die gegenseitige Anordnung der Segmente und der flexiblen Verbindungselemente derart erfolgt, dass sich eine im Wesentlichen verwindungsarme Struktur ergibt, sodass sich die mit den herkömmlichen Systemen auf Grund der Federstruktur ergebenden Nachteile vermieden werden können. Wesentlich ist hierbei, dass die einzelnen Segmente in Abstand voneinander angeordnet sind, sodass sie entsprechend der gewünschten Krümmung der flexiblen Welle gegeneinander verkippt werden können. Die vorzugsweise aus einem unnachgiebigen Material bestehenden Segmente erlauben hierbei gleichzeitig eine optimale Führung der Welle innerhalb des gekrümmten und insbesondere biegsamen Rohrs.

Die Verbindungselemente sind gemäß einer bevorzugten Weiterbildung als diskrete Verbindungselemente, das heißt von den Segmenten gesonderte Verbindungselemente ausgebildet, wobei die Verbindungselemente zwischen zwei Segmenten bzw. zu den Endstücken mit Vorteil von wenigstens zwei, vorzugsweise wenigstens drei in Abstand voneinander und vorzugsweise parallel angeordneten flexiblen Stäben, insbesondere hochelastischen Drähten, gebildet sind. Die Stäbe können hierbei als sich über die gesamte Länge der flexiblen Welle erstreckende Stäbe ausgebildet sein, die die einzelnen Segmente durchsetzen. Alternativ ist auch eine Ausbildung denkbar, bei welcher die einzelnen Stäbe jeweils nur zwei oder drei Segmente miteinander verbinden und für weitere Segmente jeweils weitere flexible Stäbe vorgesehen sind. Durch die Verwendung von flexiblen Stäben zwischen den einzelnen Segmenten und insbesondere durch die Verwendung von hochelastischen Drähten wird die erforderliche Biegsamkeit der Welle gewährleistet, wobei dadurch, dass wenigstens zwei und vorzugsweise wenigstens drei in Abstand voneinander angeordnete Stäbe vorgesehen sind, die erforderliche Torsionssteifigkeit gewährleistet ist. Die flexiblen Stäbe sollen hierbei im Querschnitt der Welle gesehen in einem derartigen Abstand voneinander angeordnet sein, dass sie sich auch bei der maximal vorgesehenen Verbiegung der Welle nicht berühren, um Reibungsverluste zu vermeiden.

Gemäß einer bevorzugten Weiterbildung ist vorgesehen, dass die Verbindungselemente bzw. Stäbe außerhalb der Wellenachse angeordnet sind, wobei weiters mit Vorteil die Verbindungselemente bzw. Stäbe im Querschnitt gesehen auf wenigstens einem zur Wellenachse konzentrischen Kreis angeordnet sind. Durch die Anordnung auf einem im Querschnitt gesehen konzentrischen Kreis werden Unwuchten vermieden, sodass hohe Drehzahlen ermöglicht werden. Dabei sind die Verbindungselemente bzw. Stäbe mit Vorteil gleichmäßig über den konzentrischen Kreis verteilt angeordnet.

Dadurch, dass die Verbindungselemente bzw. Stäbe außerhalb der Wellenachse angeordnet sind, wird die Möglichkeit geschaffen, dass die Segmente jeweils eine in der Wellenachse liegende Durchführung aufweisen. Die Durchführung erlaubt einen Medientransport durch die flexible Welle hindurch, sodass beispielsweise Kühlgas, Spülmedium, Schmiermedium, Pharmazeutika und dergleichen dem Werkzeugkopf zugeführt werden können.

Um dem Längenausgleich der Stäbe bei der Rotation Rechnung zu tragen, welche dadurch erforderlich ist, dass die Stäbe in der kurvenäußeren Position im Vergleich zur kurveninneren Position entsprechend der jeweiligen Biegung der flexiblen Welle auf größerem Radius liegen, ist die Ausbildung mit Vorteil derart weitergebildet, dass die Stäbe in jeweils wenigstens einem der beiden Endstücke in axialer Richtung verschieblich in Aufnahmebohrungen aufgenommen sind. Dadurch können die Stäbe jeweils unterschiedlich tief in die Endstücke eintauchen, sodass die Stäbe selbst nicht in Längsrichtung elastisch ausgebildet sein müssen. In diesem Zusammenhang ist es weiters vorteilhaft, wenn die Segmente mit Bohrungen zum Durchführen der Stäbe ausgebildet und auf den Stäben verschieblich geführt sind, sodass über die Länge der Stäbe keine Zugspannungen auftreten.

Um eine optimale Kraftübertragung mit möglichst geringer Reibung sicher zu stellen, ist gemäß einer bevorzugten Weiterbildung vorgesehen, dass die Segmente als scheibenförmige Segmente ausgebildet sind. Dabei ist eine Ausbildung bevorzugt, bei welcher die Segmente mit kreisförmigem Querschnitt ausgebildet und mit ihrem Mantel am Innenmantel des Rohrs geführt sind. Dadurch wird eine möglichst spielfreie Führung der flexiblen Welle im Rohr ermöglicht.

Um eine optimale Konturanpassung auch bei einer entsprechenden Krümmung des Rohres sicher zu stellen, ist die Ausbildung bevorzugt derart weitergebildet, dass die Segmente eine nach außen bombierte Mantelfläche aufweisen, wobei die Bombierung beispielsweise der maximalen Krümmung des Rohres entsprechen kann. Dadurch wird die Reibung zwischen den Segmenten zusätzlich reduziert, da an den Außenkanten der Segmente im Kontaktbereich mit dem Außenrohr keine Druckspitzen auftreten.

Um eine gleichmäßige Verteilung der Segmente über die Länge der Welle sicher zu stellen, ist die Ausbildung bevorzugt derart getroffen, dass zwischen den Segmenten jeweils wenigstens ein von den Verbindungselementen gesondertes Distanzelement angeordnet ist. Dadurch wird eine gleichmäßige Krümmung der Welle

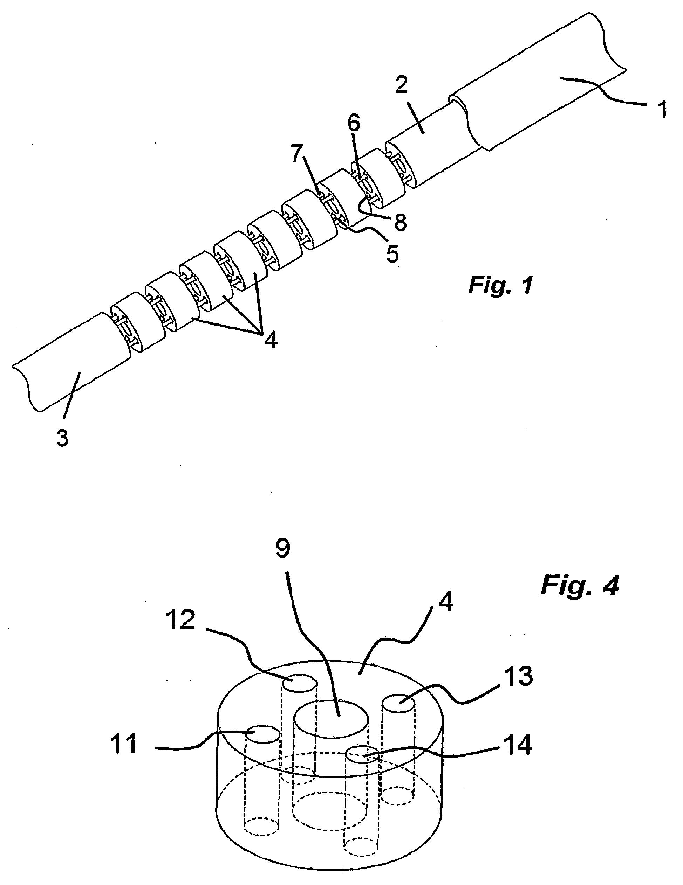

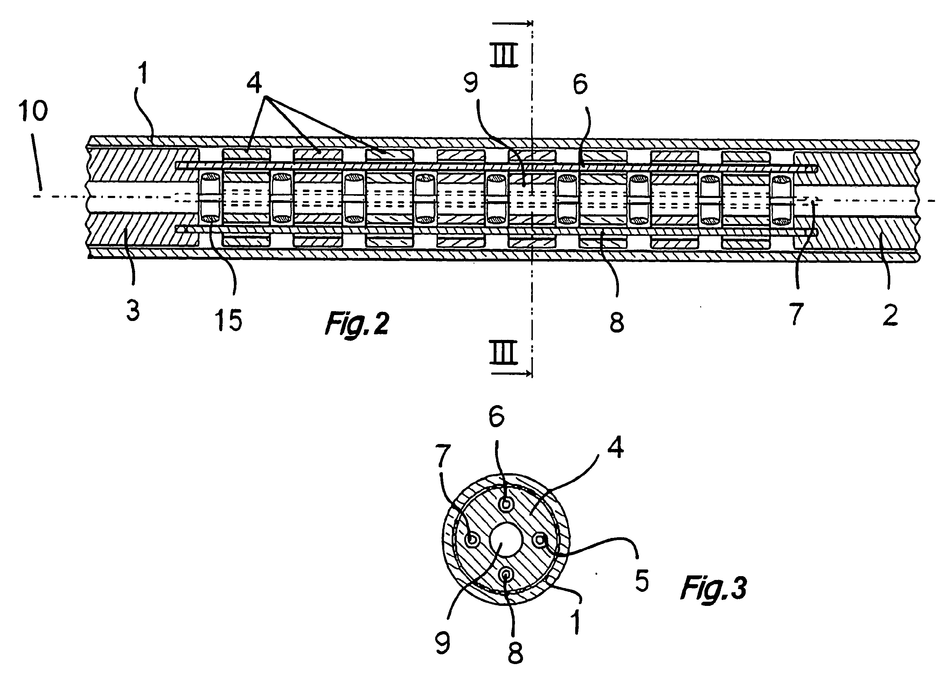

Die Erfindung wird nachfolgend anhand eines in der Zeichnung schematisch dargestellten Ausführungsbeispiels dargestellt. In dieser zeigt Fig. 1 eine perspektivische Ansicht der erfindungsgemäßen Antriebswelle, Fig. 2 einen Längsschnitt der Antriebswelle, Fig. 3 einen Querschnitt gemäß der Linie III-III der Fig. 2 und Fig. 4 eine Detailansicht eines Segments.

In Fig. 1 ist das flexible Rohr, in welchem die Antriebswelle rotierbar geführt ist, mit 1 bezeichnet. Die Antriebswelle besteht aus einem antriebsseitigen Endstück 2 und einem abtriebsseitigen Endstück 3. Zwischen den Endstücken 2 und 3 ist eine Mehrzahl von in Abstand voneinander angeordneten Segmenten 4 vorgesehen. Die Segmente 4 sind mit Hilfe von flexiblen Verbindungselementen untereinander und mit den Endstücken 2 und 3 verbunden, wobei die Verbindungselemente im vorliegenden Fall von vier flexiblen Stäben 5, 6, 7 und 8 gebildet sind.

Wie insbesondere in Fig. 2 ersichtlich ist, sind die Stäbe 5, 6, 7 und 8 als durchgehende Stäbe ausgebildet, die die beiden Endstücke 2 und 3 verbinden und entsprechende Bohrungen der Segmente 4 durchsetzen. Die Stäbe 5, 6, 7 und 8 tauchen in entsprechende Aufnahmebohrungen in den Endstücken 2 und 3 ein, über ihre gesamt Länge erzwungen, wobei zu diesem Zweck bevorzugt alle Segmente die gleiche axiale Länge aufweisen und besonders bevorzugt die Abstände zwischen den Segmenten gleich sind. Die Distanzelemente können hierbei bevorzugt als elastische Elemente ausgebildet sein, um sich an die jeweilige Krümmung der Welle anzupassen.

Die Übertragung besonders hoher Drehmomente bei gleichzeitiger Maximierung der Torsionssteifigkeit wird bevorzugt dadurch erreicht, dass die axiale Länge der Segmente größer gewählt ist als der Abstand zwischen den segmenten. Hierbei muse lediglich dafür Sorge getragen werden, dass der Abstand zwischen den Segmenten ausreicht, um die gewünschte Krümmung zuzulassen.

Die erfindungsgemäße flexible Welle kommt erfindungsgemäß in einem handgeführten Arbeitsgerät, wie z.B. in einem chirurgischen Gerät, zum Einsatz, welches mit einem Rotationsantrieb versehen ist, der mit dem antriebsseitigen Endstück der flexiblen Welle verbunden ist, wobei das abtriebsseitige Ende der Antriebswelle mit einem Rotationskopf oder dergleichen gekoppelt ist.
wobei die Stäbe in den Aufnahmebohrungen verschieblich sind, um den erforderlichen Längenausgleich bei einer Biegung der Welle zu ermöglichen. Die Stäbe 5, 6, 7 und 8 sind beispielsweise aus einer superelastischen Legierung gebildet. In Fig. 2 ist weiters ersichtlich, dass die Segmente 4 eine zentrale Durchbrechung 9 aufweisen, die zur Wellenachse 10 konzentrisch ist. Die Durchbrechungen 9 definieren eine zentrale Durchführung für Medien verschiedenster Art, wobei die Medien durch einen der Übersichtlichkeit halber nicht dargestellten flexiblen Schlauch transportiert werden können, der nicht mit der Welle mitrotiert. Die zwischen den einzelnen Segmenten 4 angeordneten Distanzelemente sind mit 15 bezeichnet.

In der Querschnittsdarstellung gemäß Fig. 3 ist die zentrale Durchführung 9 besser ersichtlich, wobei ebenfalls ersichtlich ist, dass die Stäbe 5, 6, 7 und 8 auf einem zur Wellenachse 10 konzentrischen Kreis angeordnet sind.

In der Detaildarstellung des Segments 4 in Fig. 4 sind die parallelen Bohrungen 11, 12, 13 und 14 ersichtlich, die von den Stäben 5, 6, 7 bzw. 8 durchsetzt werden.

## Patentansprüche

1. Handgeführtes chirurgisches Arbeitsgerät mit einem Rotationsantrieb, einer mit dem Rotationsantrieb verbundenen, in einem gekrümmten, vorzugsweise biegsamen Rohr rotierbar geführten flexiblen Antriebswelle und einem mit dem abtriebsseitigen Ende der Antriebswelle verbundenen Rotationskopf, wobei die flexible Antriebswelle von einem antriebsseitigen und einem abtriebsseitigen Endstück und einer Mehrzahl von zwischen den beiden Endstücken (2,3) in Abstand voneinander angeordneten Segmenten (4) gebildet ist, die mit Hilfe von flexiblen Verbindungselementen (5,6,7,8) untereinander und mit den Endstücken (2,3) zur Übertragung der Rotationsbewegung verbunden sind, **dadurch gekennzeichnet, dass** die Segmente (4) jeweils eine in der wellenachse (10) liegende Durchführung (9) aufweisen, in welcher ein flexibler, nicht mit der Welle mitrotierender Schlauch für den Transport von Medien, wie z.B. Kühlgas, Spülmedium, Schmiermedium oder Pharmazeutika angeordnet ist, wobei die Medien dem Rotationskopf zuführbar sind.

2. Arbeitsgerät nach Anspruch 1, **dadurch gekennzeichnet, dass** die Verbindungselemente (5,6,7,8) zwischen zwei Segmenten (4) bzw. zu den Endstücken (2,3) von wenigstens zwei, vorzugsweise wenigstens drei in Abstand voneinander und vorzugsweise parallel angeordneten flexiblen Stäben, insbesondere hochelastischen Drähten, gebildet sind.

3. Arbeitsgerät nach Anspruch 1 oder 2, **dadurch gekennzeichnet, dass** die Verbindungselemente bzw. Stäbe (5,6,7,8) außerhalb der Wellenachse (10) angeordnet sind.

4. Arbeitsgerät nach Anspruch 1, 2 oder 3, **dadurch gekennzeichnet, dass** die Verbindungselemente bzw. Stäbe (5,6,7,8) im Querschnitt gesehen auf wenigstens einem zur Wellenachse (10) konzentrischen Kreis angeordnet sind.

5. Arbeitsgerät nach einem der Ansprüche 1 bis 4, **dadurch gekennzeichnet, dass** die Stäbe (5,6,7,8) in jeweils wenigstens einem der beiden Endstücke (2,3) in axialer Richtung verschieblich in Aufnahmebohrungen aufgenommen sind.

6. Arbeitsgerät nach einem der Ansprüche 1 bis 5, **dadurch gekennzeichnet, dass** die Segmente (4) mit Bohrungen (11,12,13,14) zum Durchführen der Stäbe (5,6,7,8) ausgebildet und auf den Stäben verschieblich geführt sind.

7. Antriebswelle nach einem der Ansprüche 1 bis 6**, dadurch gekennzeichnet, dass** die Segmente (4) als scheibenförmige Segmente ausgebildet sind.

8. Arbeitsgerät nach einem der Ansprüche 1 bis 7, **dadurch gekennzeichnet, dass** die Segmente (4) mit kreisförmigem Querschnitt ausgebildet und mit ihrem Mantel am Innenmantel des Rohrs (1) geführt sind.

9. Antriebswelle nach einem der Ansprüche 1 bis 8, **dadurch gekennzeichnet, dass** die Segmente (4) eine nach außen bombierte Mantelfläche aufweisen.

10. Arbeitsgerät nach einem der Ansprüche 1 bis 9, **dadurch gekennzeichnet, dass** zwischen den Segmenten (4) jeweils wenigstens ein von den Verbindungselementen gesondertes Distanzelement (15) angeordnet ist.

11. Arbeitsgerät nach einem der Ansprüche 1 bis 10, **dadurch gekennzeichnet, dass** alle Segmente (4) die gleiche axiale Länge aufweisen.

12. Arbeitsgerät nach einem der Ansprüche 1 bis 11, **dadurch gekennzeichnet, dass** die axiale Länge der Segmente (4) größer gewählt ist als der Abstand zwischen den Segmenten (4).

## Claims

1. Hand-operated surgical working apparatus with a rotary drive, a flexible drive shaft connected with the rotary drive, guided rotatably in a curved, preferably bendable tube, and with a rotary head connected with the end of the drive shaft on the output side, wherein the flexible drive shaft is formed by an end piece on the driving side and an end piece on the output side and by a plurality of segments (4) arranged at a distance from each other between the two end pieces (2, 3), which segments are connected by means of flexible connecting elements (5, 6, 7, 8) with each other and with the end pieces (2, 3) for the transmission of the rotary movement, **characterized in that** the segments (4) each have an opening (9) lying in the shaft axis (10), in which a flexible tube, which is not co-rotating with the drive shaft, is arranged to transport media, such as cooling gas, flushing medium, lubricating medium or pharmaceuticals, whereby the media can be supplied to the tool head.

2. Working apparatus according to Claim 1, **characterized in that** the connecting elements (5, 6, 7, 8) between two segments (4) or to the end pieces (2, 3) respectively are formed by at least two, preferably at least three flexible rods, in particular highly elastic wires, arranged at a distance from each other and preferably parallelly.

3. Working apparatus according to Claim 1 or 2, **characterized in that** the connecting elements or rods (5, 6, 7, 8) are arranged outside the shaft axis (10).

4. Working apparatus according to Claim 1, 2 or 3, **characterized in that** the connecting elements or rods (5, 6, 7, 8), viewed in cross-section, are arranged on at least one circle concentric to the shaft axis (10).

5. Working apparatus according to any of Claims 1 to 4, **characterized in that** the rods (5, 6, 7, 8) are held in receiving bores in at least one of the two end pieces (2, 3) respectively so as to be displaceable in axial direction.

6. Working apparatus according to any of Claims 1 to 5, **characterized in that** the segments (4) are constructed with bores (11, 12, 13, 14) for the guiding through of the rods (5, 6, 7, 8) and are guided displaceably on the rods.

7. Drive shaft according to any of Claims 1 to 6, **characterized in that** the segments (4) are constructed as disc-shaped segments.

8. Working apparatus according to any of Claims 1 to 7, **characterized in that** the segments (4) are constructed with a circular cross-section and are guided with their covering surface on the inner covering surface of the tube (1).

9. Drive shaft according to any of Claims 1 to 8, **characterized in that** the segments (4) have an outwardly cambered covering surface.

10. Working apparatus according to any of Claims 1 to 9, **characterized in that** between the segments (4) respectively at least one spacer element (15) is arranged which is separate from the connecting elements.

11. Working apparatus according to any of Claims 1 to 10, **characterized in that** all the segments (4) have the same axial length.

12. Working apparatus according to any of Claims 1 to 11, **characterized in that** the axial length of the segments (4) is selected to be greater than the distance between the segments (4).

## Revendications

1. Instrument de travail chirurgical à main, comprenant un entraînement rotatif, un arbre de commande flexible, relié à l'entraînement rotatif et guidé en rotation dans un tuyau courbe de préférence souple, et une tête rotative reliée à l'extrémité côté sortie de l'arbre de commande, dans lequel l'arbre de commande flexible est constitué par un embout côté entrée et un embout côté sortie et une pluralité de segments (4) disposés entre les deux embouts (2, 3) en étant espacés les uns par rapport aux autres, lesdits segments étant reliés à l'aide d'éléments de connexion flexibles (5, 6, 7, 8) entre eux et aux embouts (2, 3) pour transmettre le mouvement rotatif, **caractérisé en ce que** les segments (4) présentent respectivement un passage (9) situé dans l'axe d'arbre (10) et dans lequel est disposé un tuyau flexible ne tournant pas avec l'arbre et destiné à transporter des produits, comme par exemple un gaz réfrigérant, un produit de rinçage, un lubrifiant ou des produits pharmaceutiques, les produits pouvant être amenés à la tête rotative.

2. Instrument de travail selon la revendication 1, **caractérisé en ce que** les éléments de connexion (5, 6, 7, 8) sont formés entre deux segments (4) ou par rapport aux embouts (2, 3) d'au moins deux, de préférence d'au moins trois, tiges flexibles, en particulier des fils métalliques hautement élastiques, disposées en étant espacées les unes par rapport aux autres et de préférence en parallèle.

3. Instrument de travail selon la revendication 1 ou 2, **caractérisé en ce que** les éléments de connexion ou tiges (5, 6, 7, 8) sont disposés en dehors de l'axe d'arbre (10).

4. Instrument de travail selon la revendication 1, 2 ou 3, **caractérisé en ce que** les éléments de connexion ou tiges (5, 6, 7, 8), vus en section transversale, sont disposés sur au moins un cercle concentrique par rapport à l'axe d'arbre (10).

5. Instrument de travail selon l'une quelconque des revendications 1 à 4, **caractérisé en ce que** les tiges (5, 6, 7, 8) sont reçues de manière mobile dans la direction axiale dans respectivement au moins l'un des deux embouts (2, 3) dans des perçages de positionnement.

6. Instrument de travail selon l'une quelconque des revendications 1 à 5, **caractérisé en ce que** les segments (4) sont réalisés avec des perçages (11, 12, 13, 14) pour faire passer les tiges (5, 6, 7, 8) et sont guidés de manière mobile sur les tiges.

7. Arbre de commande selon l'une quelconque des revendications 1 à 6, **caractérisé en ce que** les segments (4) sont réalisés comme des segments en forme de disque.

8. Instrument de travail selon l'une quelconque des revendications 1 à 7, **caractérisé en ce que** les segments (4) sont réalisés avec une section transversale circulaire et sont guidés par leur gaine au niveau de la gaine intérieure du tuyau (1).

9. Arbre de commande selon l'une quelconque des revendications 1 à 8, **caractérisé en ce que** les segments (4) présentent une surface de gaine bombée vers l'extérieur.

10. Instrument de travail selon l'une quelconque des revendications 1 à 9, **caractérisé en ce que** respectivement au moins un élément d'espacement (15) séparé des éléments de connexion est disposé entre les segments (4).

11. Instrument de travail selon l'une quelconque des revendications 1 à 10, **caractérisé en ce que** tous les segments (4) ont la même longueur axiale.

12. Instrument de travail selon l'une quelconque des revendications 1 à 11, **caractérisé en ce que** la longueur axiale des segments (4) est choisie pour être supérieure à l'espacement entre les segments (4).
